# EUROPÄISCHE PATENTANMELDUNG

(11) **EP 3 628 303 A1**
(43) Veröffentlichungstag der Anmeldung: **01.04.2020**
(21) Anmeldenummer: 19196888.2
(22) Anmeldetag: 12.09.2019
(51) Int. Cl.: A61J 1/14, A61M 5/28

(54) **PRIMÄRPACKMITTEL FÜR PHARMAZEUTISCHE SUBSTANZEN**

(30) Priorität: 28.09.2018 DE 102018124115
(71) Anmelder: SCHOTT Schweiz AG, 9001 St. Gallen (CH)
(72) Erfinder: AUERBACH, Judith, 9052 Niederteufen (CH)
(74) Vertreter: Harbach, Thomas

(57) **Zusammenfassung**

Die Erfindung betrifft ein Primärpackmittel, bevorzugt pharmazeutisches Primärpackmittel, besonders bevorzugt ein Fläschchen oder eine Karpule, zur Aufnahme einer Substanz, bevorzugt einem Pharmazeutikum, umfassend einen Hohlkörper (1), wobei der Hohlkörper (1) ein Cycloolefin-Polymer oder ein Cycloolefin-Copolymer umfasst und eine Öffnung (3) aufweist, und einen Verschluss (2), wobei der Verschluss (2) die Öffnung (3) verschließt, ein normales Elastomer und/oder ein thermoplastisches Elastomer umfasst und einen Durchstechbereich (4) zum Einführen einer Kanüle in den Hohlkörper (1) umfasst, dadurch gekennzeichnet, dass das normale Elastomer und/oder das thermoplastische Elastomer des Verschlusses (2) nicht zerstörungsfrei lösbar mit dem Hohlkörper (1) verbunden ist.

## Beschreibung

Die Erfindung betrifft ein Primärpackmittel für die Aufnahme von pharmazeutischen Substanzen, sowie ein Verfahren zur Herstellung des Primärpackmittels.

Pharmazeutische Primärpackmittel wie Fläschchen und Karpulen werden zur sterilen Aufbewahrung und partiell auch direkt bei der späteren Verabreichung von pharmazeutischen Substanzen eingesetzt. Die Entnahme der pharmazeutischen Substanz erfolgt in der Regel über eine Kanüle. Hierzu wird ein entsprechender Durchstechbereich im Verschluss des Primärpackmittels durchstochen. Während bei einem Fläschchen die pharmazeutische Substanz in der Regel zuerst mit einer Spritze entnommen wird, kann aus einer Karpule, mithilfe einer geeigneten Injektionsvorrichtung, die pharmazeutische Substanz direkt verabreicht werden. Unabhängig von der späteren Applikation ist für derartige Primärpackmittel ein dichter Verschluss, welcher Sterilität gewährleistet und gleichzeitig eine einfache Entnahme mittels einer Kanüle ermöglicht, von zentraler Bedeutung.

Pharmazeutische Primärpackmittel wie Fläschchen und Karpulen sind in der Regel gemäß Stand der Technik wie im Folgenden beschrieben ausgebildet. Die Primärpackmittel bestehen üblicherweise aus einem Hohlkörper, welcher Körper, Schulter, Hals, Bördelrand und Öffnung aufweist. Der Hohlkörper kann aus verschiedenen Materialien gefertigt werden, unter anderem Glas, Cycloolefin-Polymer oder Cycloolefin-Copolymer. Zusätzlich wird, zumeist nach dem Befüllen mit einer pharmazeutischen Substanz, ein Verschluss auf der Mündung angebracht. Der Verschluss besteht aus zwei Elementen, einem Septum und einem Krimp. Das Septum besteht in der Regel aus einem Gummi und verfügt über einen Einstechbereich. Der Krimp ist üblicherweise aus einer Aluminiumlegierung und weist eine mittige Aussparung für den Einstechbereich auf. Der Verschluss wird separat aus den beiden Elementen gefertigt. Entsprechende Verschlüsse für pharmazeutische Primärpackmittel sind hinlänglich aus dem Stand der Technik bekannt und beispielsweise durch ISO 8872:2003 reguliert. Das Septum wird durch Aufbördeln des Krimps mit dem Hohlkörper verbunden. Dabei wird das Septum durch den Krimp aufgedrückt und dadurch der Hohlkörper mit dem Verschluss formschlüssig verbunden. Die Verbindung zwischen Hohlkörper und Septum kann durch Entfernung des Krimps zerstörungsfrei und rückstandslos gelöst werden. Durch erneutes Aufbördeln eines Krimps auf den Hohlkörper kann unter Verwendung desselben Septums die Verbindung in gleicher Qualität wiederhergestellt werden.

Der Stand der Technik weist die im Folgenden geschilderten Nachteile auf. Nach der Fertigung des Hohlkörpers muss der Verschluss separat hergestellt und montiert werden. Somit werden mehrere Elemente und Fertigungsschritte zur Herstellung des Primärpackmittels benötigt, wodurch sich der Materialaufwand erhöht, die Herstellung länger und aufwendiger wird und somit auch schwieriger zu automatisieren ist. Die Verwendung verschiedener Elemente und Fertigungsschritte wirkt sich außerdem zusätzlich nachteilig aus. Zum einen ist die Wahrscheinlichkeit, das Primärpackmittel während der einzelnen Fertigungsschritte mit Partikeln zu kontaminieren, erhöht. Zum anderen birgt die separate Montage des Verschlusses das Risiko Keime einzuschließen, sodass die Dichtfläche des Verschlusses aufwendig sterilisiert werden muss. Weiterhin besteht beim Aufbördeln des Verschlusses die Gefahr, dass das Primärpackmittel nicht dicht verschlossen wird und die aufgenommene pharmazeutische Substanz entweichen kann oder Keime von außen eindringen können.

Auch die Art der Befestigung des Verschlusses an dem Hohlkörper, gemäß Stand der Technik, ist nachteilig. Zum Aufbördeln des Verschlusses muss der Hohlkörper zwingend einen Bördelrand aufweisen, dies ist beispielsweise für Karpulen genormt durch ISO 13926-1:2005-10. Deshalb sind Abweichungen von der Form des Hohlkörpers nicht oder nur begrenzt realisierbar. Somit sind Designveränderungen des Primärpackmittels, wie beispielsweise Anpassungen in der Form, Größe oder das Hinzufügen von Formschlusselementen, nicht oder nur begrenzt realisierbar.

Speziell bei Primärpackmitteln, geeignet zur direkten Verabreichung von Substanzen wie Karpulen in einer geeigneten Injektionsvorrichtung, führen Designlimitierungen in der Form zu unerwünschten Totvolumina im Bereich der Mündung. Der Kolben kann nur bis zum Beginn der Verjüngung vordringen und die verbleibende Substanz somit nicht aus dem Primärpackmittel befördern. Eine Verringerung des Totvolumens kann aufgrund der genannten Formrestriktionen nicht oder nur begrenzt erreicht werden.

Aufgabe der vorliegenden Erfindung ist es, die Nachteile des Stands der Technik zu überwinden und insbesondere, ein Primärpackmittel für pharmazeutische Substanzen bereitzustellen, dass das Risiko eines Einschlusses von Partikeln oder Keimen reduziert und den Fertigungsaufwand minimiert, sowie ein entsprechendes Herstellungsverfahren des Primärpackmittels bereitzustellen. Ferner ist es Aufgabe der Erfindung, eine erhöhte Designfreiheit in Form und Größe des Primärpackmittels zu ermöglichen.

Die Aufgabe wird gelöst durch die unabhängigen Ansprüche. Bevorzugte Ausführungsformen sind in den abhängigen Ansprüchen ausgeführt.

Das erfindungsgemäße Primärpackmittel, bevorzugt pharmazeutische Primärpackmittel, besonders bevorzugt ein Fläschchen oder eine Karpule, zur Aufnahme einer Substanz, bevorzugt einem Pharmazeutikum, umfasst einen Hohlkörper, wobei der Hohlkörper ein Cycloolefin-Polymer oder ein Cycloolefin-Copolymer umfasst und eine Öffnung aufweist, und einen Verschluss, wobei der Verschluss die Öffnung verschließt, ein normales Elastomer und/oder ein thermoplastisches Elastomer umfasst und einen Durchstechbereich zum Einführen einer Kanüle in den Hohlkörper umfasst, dadurch gekennzeichnet, dass das normale Elastomer und/oder das thermoplastische Elastomer des Verschlusses nicht zerstörungsfrei lösbar mit dem Hohlkörper verbunden ist.

Der Hohlkörper kann in Form eines Fläschchens oder einer Karpule ausgebildet sein. Fläschchen und Karpulen bilden typischerweise Körper, Schulter, Hals, Bördelrand und Öffnung aus. Der Hohlkörper kann auch einen Boden aufweisen. Der Hohlkörper kann neben Cycloolefin-Polymer oder Cycloolefin-Copolymer auch andere Materialien umfassen. Dabei können das Cycloolefin-Polymer oder das Cycloolefin-Copolymer Additive enthalten. Als Additiv wird bevorzugt ein Farbstoff verwendet. Weiterhin kann der Hohlkörper auch aus anderen Materialien gefertigt sein. Denkbar ist auch ein mehrschichtiger Aufbau des Hohlkörpers aus verschiedenen Materialien, wie beispielsweise durch ein Sandwich-Spritzgussverfahren gefertigt. Die Wandstärke des Hohlkörpers beträgt bevorzugt 0,5 mm bis 3 mm, jedoch sind auch andere Wandstärken denkbar. Die Form des Hohlkörpers ist lediglich in der Ausbildung einer Öffnung limitiert.

Durch die Öffnung kann die aufgenommene Substanz aus dem Hohlkörper entnommen werden. Zusätzlich kann der Hohlkörper eine zweite Öffnung, beispielsweise zum Befüllen, umfassen. Eine Karpule weist in der Regel eine zweite Öffnung auf. Im weiteren Verlauf wird auf die zweite Öffnung nicht weiter eingegangen und der Begriff Öffnung bezieht sich auf die Öffnung zur Entnahme, welche durch den Verschluss verschlossen wird.

Der Verschluss ist auf der Öffnung angeordnet. Dabei sind Verschluss und Hohlkörper eigenständig und ohne Hilfsmittel fest miteinander verbunden. Es kann auf einen aufgebördelten Krimp oder vergleichbare Vorrichtungen zur Befestigung verzichtet werden.

Unter einem normalen Elastomer sind chemisch vernetzte Polymere, welche nicht ohne Zersetzung wieder voneinander gelöst werden können, zu verstehen. Normale Elastomere können sich unter Zug- und Druckbelastung elastisch verformen, nehmen aber bei Wegnahme der Belastung ihre Ursprungsform wieder an. Außerdem weisen normale Elastomere unter Wärmezufuhr kein thermoplastisches Verhalten auf und sind daher nicht durch Spritzgussverfahren herstellbar oder formbar.

Unter thermoplastischen Elastomeren sind vernetzte Polymere zu verstehen, welche bei Raumtemperatur ein vergleichbares elastisches Verformungsverhalten wie normalen Elastomere aufweisen. Bei Wärmezufuhr sind thermoplastische Elastomere jedoch plastisch verformbar. Der Vorgang ist reversibel und kann durch Abkühlung und erneute Erwärmung wiederholt werden. Im Sinne dieser Anmeldung ist daher zwischen normalen Elastomeren und thermoplastischen Elastomeren zu unterscheiden. Ein thermoplastisches Elastomer kann beispielsweise ein thermoplastisches Polyolefin-Elastomer, wie ein Cycloolefin-Polymer-Elastomer oder ein Cycloolefin-Copolymer-Elastomer, sein. Ein derartiges thermoplastisches Elastomer ist unter dem Markennamen TOPAS® Elastomer E-140 erhältlich.

Der Durchstechbereich entspricht einem Teil des Gesamtvolumens des Verschlusses. Dabei ist der Durchstechbereich bevorzugt in zentraler Position im Verschluss, in Form eines vertikal ausgerichteten Zylinders angeordnet. Weiterhin bevorzugt ist der Übergang innerhalb des Verschlusses zum Durchstechbereich homogen und nicht durch eine scharfe Grenzfläche gekennzeichnet.

Die Verbindung zwischen normalem Elastomer und/oder thermoplastischem Elastomer des Verschlusses und Cycloolefin-Polymer oder Cycloolefin-Copolymer des Hohlkörpers ist erfindungsgemäß nicht zerstörungsfrei trennbar. Unter nicht zerstörungsfrei trennbar ist zu verstehen, dass nach einer Trennung von Hohlkörper und Verschluss eine erneute Verbindung desselben Verschlusses, in gleicher Qualität, entsprechend der ursprünglichen Verbindung nicht möglich ist. Außerdem kann der Verschluss nicht rückstandsfrei von dem Hohlkörper getrennt werden. Bei der Trennung werden Hohlkörper und Verschluss beschädigt, so dass Rückstände des Materials an der jeweils anderen Komponente zurückbleiben.

Das aufgenommene Pharmazeutikum kann beispielsweise eine Flüssigkeit oder ein Feststoff sein. Denkbar ist außerdem die Aufnahme einer Substanz mit einer bestimmten Dosierung eines Wirkstoffs, zur definierten und einfachen Verabreichung oder anderweitigen Anwendung.

Die Designfreiheit in der Form des Hohlkörpers und Verschlusses ist für das erfindungsgemäße Primärpackmittel deutlich erhöht. Dadurch ist ein flexibler und individueller Einsatz in verschiedensten Vorrichtungen und Verfahren möglich. Weiterhin können Designprobleme wie Totvolumina bei der Entnahme von Substanzen behoben oder zumindest reduziert werden. Das erfindungsgemäße Primärpackmittel kann in einem einzelnen Verfahren hergestellt werden. Aus diesem Grund können Materialaufwand und Herstellungsdauer minimiert werden. Ferner wird durch die Vereinfachung der Herstellung eine Kontamination des Primärpackmittels oder der Einschluss von Keimen verhindert oder zumindest reduziert.

In einer bevorzugten Ausführungsform des Primärpackmittels ist das normale Elastomer und/oder das thermoplastische Elastomer des Verschlusses stoffschlüssig und/oder mikroformschlüssig mit dem Hohlkörper verbunden.

Unter einer stoffschlüssigen Verbindung ist eine Verbindung zu verstehen, welche die verbundenen Bauteile durch atomare und/oder molekulare Kräfte zusammenhält. Die atomaren und molekularen Kräfte beinhalten nicht-kovalente Wechselwirkungen wie van-der-Waals-Wechselwirkungen. Eine stoffschlüssige Verbindung lässt sich nur nicht zerstörungsfrei trennen.

Unter einer mikroformschlüssigen Verbindung ist zu verstehen, dass die verbundenen Bauteile im mikroskopischen Längenbereich ineinandergreifen. Dabei lösen sich die Bauteile auch bei fehlender Kraftübertragung von außen oder untereinander nicht. Die Verbindung der Bauteile erfolgt insbesondere aufgrund der mikroskopischen Beschaffenheit ihrer Oberflächen. Die Oberflächenstruktur kann Poren, eine erhöhte Rauigkeit oder gezielte Materialunebenheiten bzw. -strukturierungen aufweisen. Eine mikroformschlüssige Verbindung lässt sich nur nicht zerstörungsfrei trennen.

In einer bevorzugten Ausführungsform des Primärpackmittels verschließt der Verschluss den Hohlkörper dicht, bevorzugt flüssigkeitsdicht, besonders bevorzugt gasdicht, wobei bevorzugt die Dichtigkeit nach dem Einführen und Ausführen einer Kanüle durch den Durchstechbereich bestehen bleibt.

Der dichte Verschluss verhindert, dass Substanzen oder Keime in den Innenraum des Primärpackmittels gelangen. Dadurch werden Kontaminationen des Innenraums und/oder einer aufgenommenen Substanz verhindert und die Sterilität des Primärpackmittels gewährleistet. Weiterhin verhindert der dichte Verschluss, dass eine aufgenommene Substanz aus dem Innenraum des Primärpackmittels entweicht und ermöglicht eine inerte Lagerung. Das Primärpackmittel behält diese Eigenschaften auch nach dem Einführen einer Kanüle und nimmt nur über die Kanüle eingeführte Substanzen auf. Ein dichter Verschluss kann durch das stoffschlüssige und/oder mikroformschlüssige und/oder makroformschlüssige Aufbringen eines normalen Elastomers und/oder thermoplastischen Elastomers auf die Öffnung des Hohlkörpers hergestellt werden.

In einer bevorzugten Ausführungsform des Primärpackmittels sind Hohlkörper und Verschluss derart geformt, dass Hohlkörper und Verschluss makroformschlüssig miteinander verbunden sind.

Unter einer makroformschlüssigen Verbindung ist zu verstehen, dass die verbundenen Bauteile im makroskopischen Längenbereich ineinandergreifen. Dabei lösen sich die Bauteile auch bei fehlender Kraftübertragung von außen oder untereinander nicht. Die Verbindung der Bauteile erfolgt insbesondere aufgrund makroskopischer struktureller Merkmale der Bauteile. Die Bauteile können Einkerbungen oder Auswölbungen aufweisen, welche einer Lösung der Verbindung zwischen den Bauteilen räumlich im Weg stehen. Die makroformschlüssige Verbindung kann die stoffschlüssige und/oder die mikroformschlüssige Verbindung ergänzen, um eine ausreichende Sterilbarriere zu gewährleisten.

In einer bevorzugten Ausführungsform des Primärpackmittels steht zumindest ein Teil des Verschlusses unter Druckspannung an zumindest einem Teil der Kontaktfläche zwischen Hohlkörper und Verschluss.

Die Druckspannung auf den Verschluss kann resultieren aus einer Kompression des Verschlusses während der Herstellung des Primärpackmittels. Aufgrund der elastischen Verformbarkeit des Verschlusses entsteht so eine Druckspannung an den Kontaktflächen zwischen Hohlkörper und Verschluss. Dadurch können die Verbindung und die Dichtigkeit zwischen Hohlkörper und Verschluss verstärkt werden.

In einer bevorzugten Ausführungsform des Primärpackmittels weist der Verschluss im Durchstechbereich eine Shore-Härte Shore-A von 20 bis 70 auf, wobei die Last maximal 30 N, bevorzugt maximal 20 N, besonders bevorzugt maximal 10 N, beträgt.

Die Shore-Härte ist ein Maß für die Härte von Materialien. Für klassische und thermoplastische Elastomere wird die Shore-Härte anhand der Eindringtiefe eines federbelasteten Stahlstifts bestimmt. Die Bestimmung der Shore-Härte für Shore-A kann an Hand der ISO 868:2003-10 für eine Probendicke von 4 mm, mit einer Haltezeit von 3 s oder 15 s und einer Last von 12,5 N erfolgen.

In einer bevorzugten Ausführungsform des Primärpackmittels umfasst der Hohlkörper an seiner Außenseite mindestens ein Formschlusselement zur formschlüssigen Aufnahme des Primärpackmittels in einer Vorrichtung.

Ein Formschlusselement ist insbesondere dann von Bedeutung, wenn das Primärpackmittel keinen klassischen Flansch ausweist. Das Formschlusselement kann in Form einer Einkerbung oder Auswölbung an der Außenseite des Hohlkörpers angeordnet sein. Bevorzugt ist das Formschlusselement am Körper des Hohlkörpers angeordnet. Es kann ein einzelnes oder eine Vielzahl von Formschlusselementen vorliegen. Das Formschlusselement besteht bevorzugt aus demselben Material wie der Hohlkörper und ist bevorzugt makroformschlüssig und/oder mikroformschlüssig und/oder stoffschlüssig mit diesem verbunden, bzw. einstückig mit dem Hohlkörper ausgebildet.

In einer bevorzugten Ausführungsform des Primärpackmittels bedeckt der Verschluss zumindest Teile der Außenseite des Hohlkörpers.

Das Bedecken der Oberfläche mit dem Verschlussmaterial kann unter anderem die Griffigkeit des Primärpackmittels erhöhen oder die Verbindung mit einer Vorrichtung verstärken. Je nach Applikation des Primärpackmittels kann der Verschluss verschiedene Teile der Außenseite des Hohlkörpers bedecken. Dabei ist auch denkbar, dass Teile der Außenseite des Hohlkörpers durch separates Verschlussmaterial bedeckt werden, welches nicht mit dem eigentlichen Verschluss verbunden ist.

In einer bevorzugten Ausführungsform des Primärpackmittels umfasst der Verschluss mindestens eine erste Schicht, umfassend ein normales Elastomer oder ein thermoplastisches Elastomer, und mindestens eine zweite Schicht, umfassend ein normales Elastomer oder ein thermoplastisches Elastomer. Dabei können die Schichten makroformschlüssig und/oder mikroformschlüssig und/oder stoffschlüssig verbunden sein.

Ein mehrschichtiger Verschluss ermöglicht die Kombination verschiedener Materialien. Dabei können für die verschiedenen Schichten materialspezifische Eigenschaften vorteilhaft ausgenutzt werden. Es kann beispielweise die Schicht, welche im Kontakt mit der aufgenommenen Substanz steht, aus einem der aufgenommen Substanz gegenüber chemisch inerten thermoplastischen Elastomer bestehen. Eine weitere angrenzende Schicht kann aus einem normalen Elastomer bestehen, welches eine erhöhte Dichtigkeit des Primärpackmittels garantiert. Für einen Verschluss aus mehreren Schichten sind verschiedene Materialkombinationen und Schichtanzahlen dankbar, welche sich unterschiedliche vorteilhafte Materialeigenschaften zu Nutzen machen. Durch die Verwendung mehrerer Schichten können zudem die Materialkosten gesenkt werden.

In einer bevorzugten Ausführungsform des Primärpackmittels enthält das Primärpackmittel eine Substanz, bevorzugt eine Substanz für therapeutische und/oder diagnostische Zwecke, besonders bevorzugt eine einzelne Dosis der Substanz.

Das Primärpackmittel kann eine Substanz wie einen Wirkstoff oder ein Pharmazeutikum enthalten. Bevorzugt enthält das Primärpackmittel eine bestimmte Dosis des Wirkstoffs oder des Pharmazeutikums. Dadurch wird die Dosierung oder direkte Verabreichung deutlich vereinfacht.

In einer weiteren bevorzugten Ausführungsform des Primärpackmittels besteht zwischen Verschluss und Hohlkörper, ein Spaltmaß im molekularen Größenbereich, bevorzugt besteht kein Spalt.

Ein Spalt zwischen Verschluss und Hohlkörper kann bei einer mikroformschlüssigen Verbindung vorliegen. Der Spalt ist jedoch, abhängig vom Material des Verschlusses und des Hohlkörpers, auf die Größenordnung einzelner Moleküle oder Atome beschränkt.

In einer weiteren bevorzugten Ausführungsform des Primärpackmittels ist der Hohlkörper entsprechend ISO 13926-1 :2005-10 ausgebildet, bevorzugt mit einem Winkel zwischen Schulter und Hals von ungefähr 30°, einem Winkel zwischen Hals und Flansch von 10° bis 20° und einem Winkel zwischen Flansch und Öffnung von 8° bis 14°.

In einer weiteren bevorzugten Ausführungsform des Primärpackmittels ist der Verschluss entsprechend ISO 8872:2003 ausgebildet, bevorzugt mit einer Zugfestigkeit von 100 N/mm² bis 180 N/mm² und einer Bruchdehnung von mindestens 80 N/mm².

In einer weiteren bevorzugten Ausführungsform des Primärpackmittels ist der Hohlkörper entsprechend ISO 11040-1:2015(E) ausgebildet, bevorzugt mit einem Winkel zwischen Schulter und Hals von 35° bis 45°, einem Winkel zwischen Hals und Flansch von 10° bis 20° und einem Winkel zwischen Flansch und Mündung von 9° bis 13°.

In einer weiteren bevorzugten Ausführungsform des Primärpackmittels ist der Verschluss entsprechend ISO 11040-3:2012(E) ausgebildet, bevorzugt mit einer Höhe von 1,3 mm bis 1,5 mm oder 1,45 mm bis 1,95 mm, einem Durchmesser von 7,5 mm und einem Durchmesser des Durchstechbereichs (8) von 3,0 mm.

Die Ausbildung des Hohlkörpers und des Verschlusses gemäß den oben genannten Normen birgt den Vorteil, dass das erfindungsgemäße Primärpackmittel in bestehenden und etablierten Vorrichtungen und Verfahren eingesetzten werden kann.

Die Erfindung umfasst ferner ein Verfahren zur Herstellung eines Primärpackmittels umfassend folgende Schritte:
- Schritt a: optionales Bereitstellen zumindest einer der Komponenten, ausgewählt aus Hohlkörper und zumindest einem Teil des Verschlusses
- Schritt b:Herstellen der restlichen Komponenten, ausgewählt aus Hohlkörper und zumindest einem Teil des Verschlusses, im Spritzgussverfahren, so dass der Verschluss die Öffnung verschließt und eine nicht zerstörungsfrei lösbare Verbindung zwischen Verschluss und Hohlkörper entsteht.

In einer bevorzugten Ausführungsform des Verfahrens wird in Schritt a der Verschluss bereitgestellt und in Schritt b wird der Hohlkörper im Spritzgussverfahren hergestellt, wobei ein Cycloolefin-Polymer oder ein Cycloolefin-Copolymer eingesetzt wird, wobei der Verschluss umspritzt wird, so dass bevorzugt Verschluss und Hohlkörper makroformschlüssig verbunden werden.

Zur Herstellung des Primärpackmittels ist die Vorlage des Verschlusses, durch Spritzguss oder anderweitig gefertigt, möglich. Es kann auch ein mehrteiliger Verschluss vorgelegt werden. Der vorgelegte Verschluss wird, bevorzugt mit einem Cycloolefin-Polymer oder einem Cycloolefin-Copolymer, umspritzt und dabei der Hohlkörper ausgebildet.

In einer bevorzugten Ausführungsform des Verfahrens wird in Schritt a der Hohlkörper mit der Öffnung bereitgestellt, optional zumindest ein Teil des Verschlusses auf die Öffnung aufgelegt und in Schritt b im Spritzguss mit einem thermoplastischen Elastomer der Verschluss vollständig ausgebildet, wobei bevorzugt Verschluss und Hohlkörper stoffschlüssig und/oder mikroformschlüssig verbunden werden.

Zur Herstellung des Primärpackmittels ist die Vorlage des Hohlkörpers, durch Spritzguss oder anderweitig gefertigt, möglich. Zusätzlich kann ein erster Teil des Verschlusses, bevorzugt ein normales Elastomer, vorgelegt werden. Die vorgelegten Teile werden, bevorzugt mit einem thermoplastischen Elastomer, umspritzt und dabei der zweite Teil des Verschlusses ausgebildet. Weiterhin kann der vorgelegte Hohlkörper auch ohne Vorlage eines Teils des Verschlusses umspritzt werden. Der vorgelegte Hohlkörper wird dann, bevorzugt mit einem thermoplastischen Elastomer, umspritzt und dabei der gesamte Verschluss ausgebildet.

In einer bevorzugten Ausführungsform des Verfahrens wird in Schritt a optional zumindest ein Teil des Verschlusses bereitgestellt und in Schritt b im Mehrkomponenten-Spritzguß werden ein Cycloolefin-Polymer oder ein Cycloolefin-Copolymer zur Herstellung des Hohlkörpers und ein thermoplastisches Elastomer zur vollständigen Ausbildung des Verschlusses gespritzt, wobei bevorzugt Verschluss und Hohlkörper makroformschlüssig und/oder stoffschlüssig und/oder mikroformschlüssig verbunden werden.

Bevorzugt wird das gesamte Primärpackmittel, also Hohlkörper und Verschluss, durch Mehrkomponenten-Spritzgießen gesamtheitlich hergestellt. Beim Mehrkomponenten-Spritzgießen kann ein Spritzgussteil aus zwei oder mehr unterschiedlichen Kunststoffen hergestellt werden. Mit Mehrkomponenten-Spritzgießverfahren ist es möglich, verschiedene Kunststoffe aneinander anzuspritzen und so eine physikalische Anbindung zwischen den Kunststoffen zu schaffen. Eine Spritzgießmaschine für Mehrkomponenten-Spritzgießen umfasst in der Regel zwei oder mehr Spritzeinheiten und nur eine Schließeinheit. Weiterhin kann ein vorgespritztes oder anderweitig vorgefertigtes Teil zum Umspritzen eingelegt werden. Es sind unterschiedliche Verfahren des Mehrkomponenten-Spritzgießens denkbar. So ist unter anderem auch ein Mehrkomponenten-Verfahren mit scharf getrennten Komponenten durchführbar. Es wird zunächst ein Vorspritzling erzeugt und anschließend mit einer anderen Komponente überspritzt. Um Platz für die zweite Komponente zu schaffen, sind unterschiedliche Verfahren wie die Umsetztechnik, die Dreh-/Verschiebetechnik oder Kernrückzugstechnik denkbar. Mehrkomponenten-Spritzgießverfahren ermöglichen eine kostengünstige Herstellung mit nur einem Werkzeug in einem Arbeitsgang. Zusätzlich sind, im Vergleich zu einer herkömmlichen Montage von Einzelkomponenten, geringe Lagetoleranzen umsetzbar. Weiterhin wird das Risiko einer Kontamination minimiert.

In einer bevorzugten Ausführungsform des Verfahrens wird zumindest ein Teil des Verschlusses während oder vor dem Spritzgussverfahren unter Druckspannung gesetzt, so dass die Druckspannung nach Abkühlung des spritzgegossenen Materials erhalten bleibt.

Der Verschluss erfährt im Spritzguss während des Umspritzens oder Anspritzens eine Kompression. Die Kompression kann durch ein Bauteil, welches einen Druck auf den Verschluss ausübt, bewirkt werden. Bevorzugt wird das druckausübende Bauteil vor Abschluss des Spritzgusses wieder entfernt, unter Aufrechterhaltung der Druckspannung auf den Verschluss.

In einer bevorzugten Ausführungsform des Verfahrens wird zumindest ein Teil der Kontaktflächen zwischen Hohlkörper und Verschluss vorbehandelt, bevorzugt durch Aktivierung, Aufrauhung oder Strukturierung zumindest einer Oberfläche, bevorzugt beider Oberflächen.

Die Aktivierung der Oberfläche kann unter anderem durch eine Flamme, ein Plasma oder eine Corona erfolgen. Eine Aufrauhung der Oberfläche kann mechanisch oder chemisch durchgeführt werden. Die Strukturierung der Oberfläche kann Muster wie Rillen und Gitter, eine chemische Funktionalisierung oder Coatings beinhalten. Dabei können sowohl nur die Oberfläche der Kontaktseite des Hohlkörpers oder des Verschlusses oder beide Oberflächen vorbehandelt werden.

### Bezugszeichenliste

- 1: Hohlkörper
- 2: Verschluss
- 2a: untere Verschlussschicht
- 2b: obere Verschlussschicht
- 2c: seitliche Verschlussschicht
- 3: Öffnung
- 4: Durchstechbereich
- 5: Formschlusselement
- 6: Körper
- 7: Schulter
- 8: Hals
- 9: Bördelrand

### Figurenbeschreibung

Fig. 1: Querschnittansicht einer ersten Ausführungsform des erfindungsgemäßen Primärpackmittels.
Fig. 2: Querschnittansicht einer zweiten Ausführungsform des erfindungsgemäßen Primärpackmittels.
Fig. 3: Querschnittansicht einer dritten Ausführungsform des erfindungsgemäßen Primärpackmittels.
Fig. 4: Querschnittansicht einer vierten Ausführungsform des erfindungsgemäßen Primärpackmittels.
Fig. 5a: Schematische Darstellung einer ersten Ausführungsform des Verschlusses mit Durchstechbereich.
Fig. 5b: Schematische Darstellung einer zweiten Ausführungsform des Verschlusses mit Durchstechbereich.

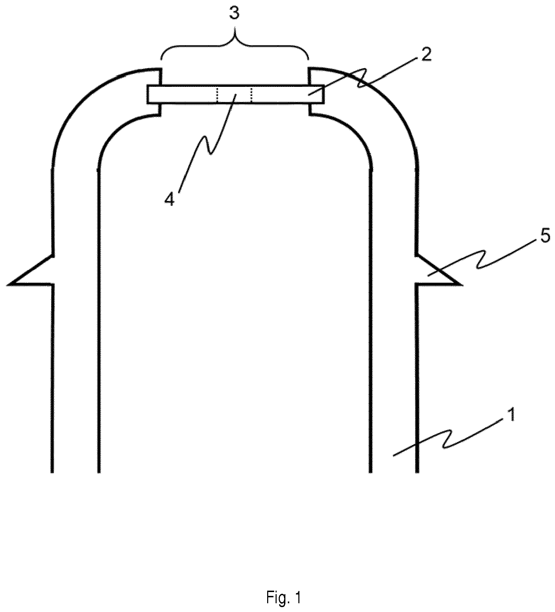

Figur 1 zeigt eine Querschnittansicht durch eine erste bevorzugte Ausführungsform des erfindungsgemäßen Primärpackmittels. Der Hohlkörper (1) umfasst an seiner Außenseite ein umlaufendes Formschlusselement (5). Die Öffnung (3) des Hohlkörpers (1) wird durch den Verschluss (2) verschlossen. Hohlkörper (1) und Verschluss (2) sind makroformschlüssig verbunden. Der Verschluss (2) greift in eine Einkerbung an der Innenseite des Hohlkörpers (1) im Bereich der Mündung (2). Der Verschluss (2) umfasst mittig ein durchgehendes Volumenelement, welches den Durchstechbereich (4) ausbildet. Der Hohlkörper (1) besteht aus Cycloolefin-Polymer oder Cycloolefin-Copolymer und der Verschluss (2) aus einem normalen Elastomer oder einem thermoplastischen Elastomer. Die Wandstärke des Hohlkörpers (1) beträgt 0,5 mm bis 3 mm. Zur Herstellung der ersten bevorzugten Ausführungsform wird der Verschluss (2) bereitgestellt und im Spritzguss mit einem Cycloolefin-Polymer oder einem Cycloolefin-Copolymer zur Ausbildung des Hohlkörpers (1) umspritzt.

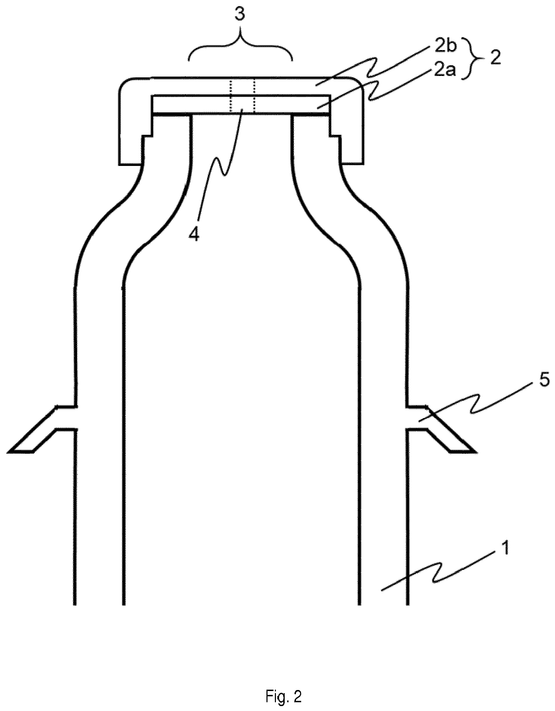

Figur 2 zeigt eine Querschnittansicht durch eine zweite bevorzugte Ausführungsform des erfindungsgemäßen Primärpackmittels. Der Hohlkörper (1) umfasst an seiner Außenseite mindestens zwei gewinkelte Formschlusselemente (5). Die Öffnung (3) des Hohlkörpers (1) wird durch den Verschluss (2) verschlossen. Der Verschluss (2) umfasst eine untere (2a) und eine obere (2b) Verschlussschicht. Die untere Verschlussschicht (2a) wird mikroformschlüssig und makroformschlüssig von der oberen Verschlussschicht (2b) auf der Öffnung (3) gehalten. Die untere Verschlussschicht (2a) kann unter einer axialen Druckspannung an den Kontaktflächen zum Hohlkörper (1) und der oberen Verschlussschicht (2b) stehen. Die obere Verschlussschicht (2b) und der Hohlkörper (1) sind stoffschlüssig und/oder mikroformschlüssig verbunden. Der Hohlkörper (1) umfasst eine Einkerbung an der Außenseite im Bereich der Öffnung (3), um die Kontaktfläche mit der oberen Verschlussschicht (2b) zu erhöhen. Der Verschluss (2) umfasst mittig ein durch untere (2a) und obere (2b) Verschlussschicht gehendes Volumenelement, welches den Durchstechbereich (4) ausbildet. Der Hohlkörper (1) besteht aus Cycloolefin-Polymer oder Cycloolefin-Copolymer. Die untere Verschlussschicht (2a) besteht aus einem normalen Elastomer und die obere Verschlussschicht (2b) besteht aus einem thermoplastischen Elastomer. Die Wandstärke des Hohlkörpers (1) beträgt 0,5 mm bis 3 mm. Zur Herstellung der zweiten bevorzugten Ausführungsform wird die untere Verschlussschicht (2a) bereitgestellt und im Mehrkomponenten-Spritzguß ein Cycloolefin-Polymer oder ein Cycloolefin-Copolymer zur Ausbildung des Hohlkörpers (1) und ein thermoplastisches Elastomer zur Ausbildung der oberen Verschlussschicht (2b) gespritzt. Alternativ wird zur Herstellung der zweiten bevorzugten Ausführungsform der Hohlkörper (1) mit der Öffnung (3) bereitgestellt, die untere Verschlussschicht (2a) bereitgestellt und auf die Öffnung (3) aufgelegt und im Spritzguss mit einem thermoplastischen Elastomer die obere Verschlussschicht (2b) ausgebildet.

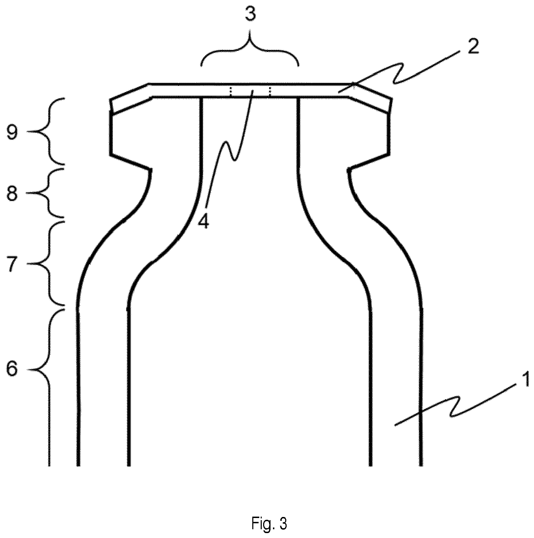

Figur 3 zeigt eine Querschnittansicht durch eine dritte bevorzugte Ausführungsform des erfindungsgemäßen Primärpackmittels. Der Hohlkörper (1) umfasst Körper (6), Schulter (7), Hals (8) und Bördelrand (9). Die Öffnung (3) des Hohlkörpers (1) wird durch den Verschluss (2) verschlossen. Hohlkörper (1) und Verschluss (2) sind stoffschlüssig und/oder mikroformschlüssig verbunden. Der Verschluss (2) umfasst mittig ein durchgehendes Volumenelement, welches den Durchstechbereich (4) ausbildet. Der Hohlkörper (1) besteht aus Cycloolefin-Polymer oder Cycloolefin-Copolymer und der Verschluss (2) aus einem thermoplastischen Elastomer. Die Wandstärke des Hohlkörpers (1) beträgt 0,5 mm bis 3 mm. Zur Herstellung der dritten bevorzugten Ausführungsform wird im Mehrkomponenten-Spritzguß ein Cycloolefin-Polymer oder ein Cycloolefin-Copolymer zur Herstellung des Hohlkörpers (1) und ein thermoplastisches Elastomer zur Ausbildung des Verschlusses (2) gespritzt.

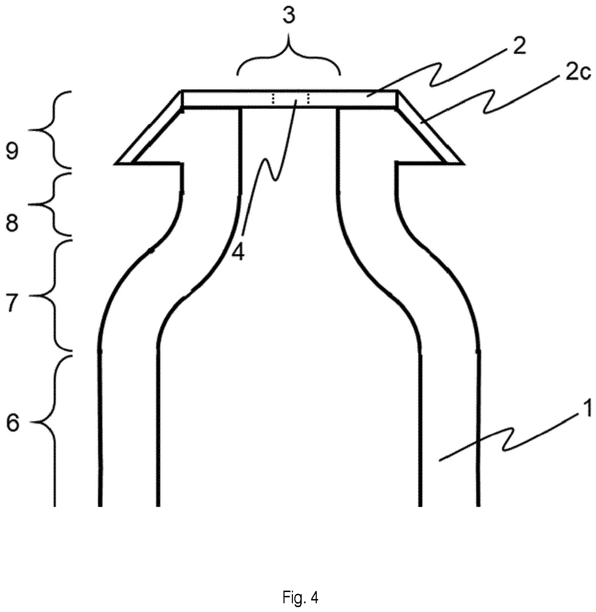

Figur 4 zeigt eine Querschnittansicht durch eine vierte bevorzugte Ausführungsform des erfindungsgemäßen Primärpackmittels. Der Hohlkörper (1) umfasst Körper (6), Schulter (7), Hals (8) und Bördelrand (9). Die Öffnung (3) des Hohlkörpers (1) wird durch den Verschluss (2) verschlossen. Hohlkörper (1) und Verschluss (2) sind stoffschlüssig und/oder mikroformschlüssig verbunden. Der Verschluss weist eine seitliche Verschlussschicht (2c) auf, welche zumindest einen Teil des Bördelrands (9) bedeckt. Die seitliche Verschlussschicht (2c) erhöht die Griffigkeit des Primärpackmittels. Der Verschluss (2) umfasst mittig ein durchgehendes Volumenelement, welches den Durchstechbereich (4) ausbildet. Der Hohlkörper (1) besteht aus Cycloolefin-Polymer oder Cycloolefin-Copolymer und der Verschluss (2) sowie die seitliche Verschlussschicht (2c) aus einem thermoplastischen Elastomer. Die Wandstärke des Hohlkörpers (1) beträgt 0,5 mm bis 3 mm. Zur Herstellung der vierten bevorzugten Ausführungsform wird der Hohlkörper (1) mit der Öffnung (3) bereitgestellt und im Spritzguss mit einem thermoplastischen Elastomer der Verschluss (2) und die seitliche Verschlussschicht (2c) ausgebildet.

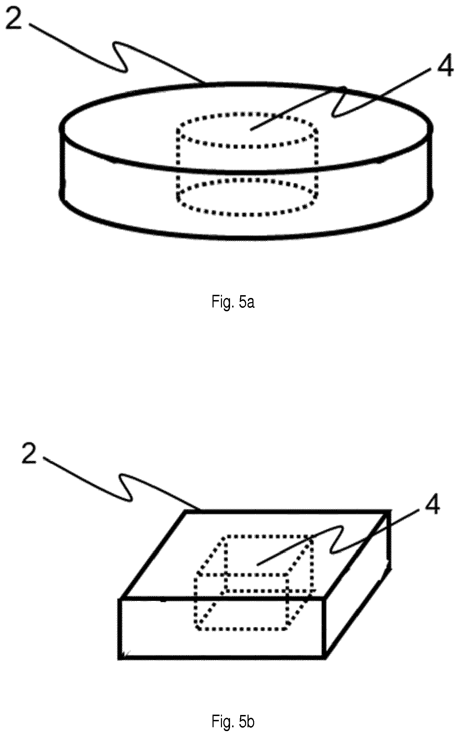

Die Figuren 5a und 5b zeigen jeweils eine schematische Darstellung des Verschlusses (2). Der Durchstechbereich (4) ist als ein Teilvolumen vom Verschluss (2) ausgebildet. Der Durchstechbereich (2) reicht von der Oberseite bis zur Unterseite des Verschlusses (2). Dies ermöglicht das Einführen einer Kanüle in den Hohlkörper des Primärpackmittels. Der Verschluss kann verschieden ausgebildet sein. Die durch Figur 5a und Figur 5b exemplarisch dargestellten Formen sind nicht limitierend zu verstehen. Vielmehr richtet sich die Form nach der Beschaffenheit und Ausbildung des Hohlkörpers im Bereich der Öffnung. Gleiches gilt für den Durchstechbereich (4). Dieser kann in verschiedenen geometrischen Formen ausgebildet sein. Bevorzugt umfasst der Durchstechbereich einen homogenen Übergang und keine scharfe Grenzfläche zum restlichen Teil des Verschlusses (2).

Das erfindungsgemäße Primärpackmittel unterscheidet sich grundlegend von den bisher verwendeten Primärpackmitteln mit einem Verschluss aus Septum und Aluminiumkrimp. Aufgrund der erfindungsgemäßen Verbindung zwischen Hohlkörper und Verschluss kann die Form des Hohlkörpers variabel der entsprechenden Applikation angepasst werden. So können beispielsweise Totvolumina im Bereich der Öffnung von Karpulen und die daraus resultierenden Verluste bei der Entnahme der aufgenommenen Substanz ausgeschlossen oder zumindest minimiert werden.

Weiterhin ist die Fertigung des erfindungsgemäßen Primärpackmittels weniger komplex und aufwendig im Vergleich zu den bisher verwendeten Primärpackmitteln. Die Anzahl der benötigten Bauteile ist geringer und das erfindungsgemäße Primärpackmittel kann in einem Prozess im Spritzguss gefertigt werden. Bei der Fertigung in einem Prozessschritt wird das Risiko, Fremdkörper oder Keime einzuschließen, ausgeschlossen oder zumindest reduziert. Insgesamt können dadurch die Herstellungskosten gesenkt werden.

## Patentansprüche

1. Primärpackmittel, bevorzugt pharmazeutisches Primärpackmittel, besonders bevorzugt ein Fläschchen oder eine Karpule, zur Aufnahme einer Substanz, bevorzugt einem Pharmazeutikum, umfassend
einen Hohlkörper (1),
wobei der Hohlkörper (1) ein Cycloolefin-Polymer oder ein Cycloolefin-Copolymer umfasst und eine Öffnung (3) aufweist,
und einen Verschluss (2),
wobei der Verschluss (2) die Öffnung (3) verschließt, ein normales Elastomer und/oder ein thermoplastisches Elastomer umfasst und
einen Durchstechbereich (4) zum Einführen einer Kanüle in den Hohlkörper (1) umfasst, **dadurch gekennzeichnet,**
**dass** das normale Elastomer und/oder das thermoplastische Elastomer des Verschlusses (2) nicht zerstörungsfrei lösbar mit dem Hohlkörper (1) verbunden ist.

2. Primärpackmittel gemäß Anspruch 1,
wobei das normale Elastomer und/oder das thermoplastische Elastomer des Verschlusses (2) stoffschlüssig und/oder mikroformschlüssig mit dem Hohlkörper (1) verbunden ist.

3. Primärpackmittel gemäß einem der vorhergehenden Ansprüche,
wobei der Verschluss (2) den Hohlkörper (1) dicht, bevorzugt flüssigkeitsdicht, besonders bevorzugt gasdicht, verschließt,
wobei bevorzugt die Dichtigkeit nach dem Einführen und Ausführen einer Kanüle durch den Durchstechbereich (4) bestehen bleibt.

4. Primärpackmittel gemäß einem der vorhergehenden Ansprüche,
wobei Hohlkörper (1) und Verschluss (2) derart geformt sind, dass Hohlkörper (1) und Verschluss (2) makroformschlüssig miteinander verbunden sind.

5. Primärpackmittel gemäß einem der vorhergehenden Ansprüche,
wobei zumindest ein Teil des Verschlusses (2) unter Druckspannung an zumindest einem Teil der Kontaktfläche zwischen Hohlkörper (1) und Verschluss (2) steht.

6. Primärpackmittel gemäß einem der vorhergehenden Ansprüche,
wobei der Verschluss im Durchstechbereich (4) eine Shore-Härte Shore-A von 20 bis 70 aufweist,
wobei die Last maximal 30 N, bevorzugt maximal 20 N, besonders bevorzugt maximal 10 N, beträgt.

7. Primärpackmittel gemäß einem der vorhergehenden Ansprüche,
wobei der Hohlkörper (1) an seiner Außenseite mindestens ein Formschlusselement (5) zur formschlüssigen Aufnahme des Primärpackmittels in einer Vorrichtung umfasst.

8. Primärpackmittel gemäß einem der vorhergehenden Ansprüche,
wobei der Verschluss (2) zumindest Teile der Außenseite des Hohlkörpers (1) bedeckt.

9. Primärpackmittel gemäß einem der vorhergehenden Ansprüche,
wobei der Verschluss (2) mindestens eine erste Schicht,
umfassend ein normales Elastomer oder ein thermoplastisches Elastomer, und mindestens eine zweite Schicht,
umfassend ein normales Elastomer oder ein thermoplastisches Elastomer,
umfasst.

10. Primärpackmittel gemäß einem der vorhergehenden Ansprüche,
wobei das Primärpackmittel eine Substanz, bevorzugt eine Substanz für therapeutische und/oder diagnostische Zwecke, besonders bevorzugt eine einzelne Dosis der Substanz, enthält.

11. Verfahren zur Herstellung eines Primärpackmittels gemäß einem der vorhergehenden Ansprüche, umfassend folgende Schritte
- Schritt a: optionales Bereitstellen zumindest einer der Komponenten, ausgewählt aus Hohlkörper (1) und zumindest einem Teil des Verschlusses (2)
- Schritt b: Herstellen der restlichen Komponenten, ausgewählt aus Hohlkörper (1) und zumindest einem Teil des Verschlusses (2), im Spritzgussverfahren,
so dass der Verschluss (2) die Öffnung (3) verschließt und
eine nicht zerstörungsfrei lösbare Verbindung zwischen Verschluss (2) und Hohlkörper (1) entsteht.

12. Verfahren gemäß Anspruch 11,
wobei in Schritt a der Verschluss (2) bereitgestellt wird und
in Schritt b der Hohlkörper (1) im Spritzgussverfahren hergestellt wird,
wobei ein Cycloolefin-Polymer oder ein Cycloolefin-Copolymer eingesetzt wird,
wobei der Verschluss (2) umspritzt wird,
so dass bevorzugt Verschluss (2) und Hohlkörper (1) makroformschlüssig verbunden werden.

13. Verfahren gemäß Anspruch 11,
wobei in Schritt a der Hohlkörper (1) mit der Öffnung (3) bereitgestellt wird,
optional zumindest ein Teil des Verschlusses (2) auf die Öffnung (3) aufgelegt wird und
in Schritt b im Spritzguss mit einem thermoplastisches Elastomer der Verschluss (2) vollständig ausgebildet wird,
wobei bevorzugt Verschluss (2) und Hohlkörper (1) stoffschlüssig und/oder mikroformschlüssig verbunden werden.

14. Verfahren gemäß Anspruch 11,
wobei in Schritt a optional zumindest ein Teil des Verschlusses (2) bereitgestellt wird und
in Schritt b im Mehrkomponenten-Spritzguß ein Cycloolefin-Polymer oder ein Cycloolefin-Copolymer zur Herstellung des Hohlkörpers (1) und ein thermoplastisches Elastomer zur vollständigen Ausbildung des Verschlusses (2) gespritzt werden,
wobei bevorzugt Verschluss (2) und Hohlkörper (1) makroformschlüssig und/oder stoffschlüssig und/oder mikroformschlüssig verbunden werden.

15. Verfahren gemäß einem der Ansprüche 11 bis 14,
wobei zumindest ein Teil des Verschlusses (2) während oder vor dem Spritzgussverfahren unter Druckspannung gesetzt wird, so dass die Druckspannung nach Abkühlung des spritzgegossenen Materials erhalten bleibt.

16. Verfahren gemäß einem der Ansprüche 11 bis 15,
wobei zumindest ein Teil der Kontaktflächen zwischen Hohlkörper (1) und Verschluss (2) vorbehandelt wird, bevorzugt durch Aktivierung, Aufrauhung oder Strukturierung zumindest einer Oberfläche, bevorzugt beider Oberflächen.
